# EUROPEAN PATENT APPLICATION

(11) **EP 4 631 534 A1**
(43) Date of publication of application: **15.10.2025**
(21) Application number: 24169556.8
(22) Date of filing: 10.04.2024
(51) Int. Cl.: A61L 26/00

(54) **ADVANCED DRESSING IN THE FORM OF A POWDER OF FUNCTIONALIZED POLYMERIC MATERIAL FOR THE RTREATMENT OF SKIN WOUNDS**

(71) Applicant: DEALFA S.R.L., 20124 Milano (IT)
(72) Inventor: PIETRA, Daniele, I-55100 Lucca (IT); BORGHINI, Alice, I-55100 Lucca (IT)
(74) Representative: Long, Giorgio

(57) **Abstract**

The present invention relates to an advanced dressing characterized by a powder of polymeric material based on poly(lactic-co-glycolic) acid, functionalized with dextran, maltodextrin, collagen, citric acid, chitosan, polyphenols, colloidal silver, hyaluronic acid and/or salts thereof, polyethylene glycol and alginate. The present invention further relates to a process for producing the powder and the use of the powder in treating non-necrotic skin wounds.

## Description

### FIELD OF THE INVENTION

The present invention relates to an advanced dressing, its use in treating non-necrotic skin wounds and a method for its production.

### BACKGROUND OF THE INVENTION

A product which promotes the tissue repair process, protecting the wound from infections and maintaining optimal moisture and oxygen permeability conditions in the wound microenvironment is an advanced dressing.

Advanced dressings can be divided into:
PASSIVE - They serve to absorb exudates and protect the lesion from external agents;
INTERACTIVE OR ACTIVE - They play an active role in tissue repair, regulating the lesion microenvironment and ensuring the ideal characteristics for the repair process to be facilitated;
BIOACTIVE - They interact with the repair processes, releasing or forming substances that directly act on the healing processes.

In the presence of certain skin lesions, the application of advanced dressings that promote their healing can be useful. Lesions which may benefit from the application of advanced dressings are wounds, ulcers and skin sores, such as diabetic ulcers, pressure ulcers and sores (bedsores), general skin ulcers and sores, surgical wounds, cut wounds, puncture wounds, gunshot wounds, abrasions, excoriations, burns.

In particular, **pressure ulcers** are areas of skin tissue damage and/or damage of the underlying tissues primarily caused from pressure, stretching or friction. This type of damage (also defined as pressure ulcer, bedsore, or decubitus ulcer or lesion), although largely preventable, constitutes an important phenomenon in hospital wards and community settings, both in terms of number of patients involved, and the time and resources required for the treatment of the problem.

**Vascular lesions of the lower limbs** are defined as those skin wounds with venous, arterial, and/or mixed vascular aetiology, located from below the knee to the foot and lasting at least eight/ten weeks. Chronic leg ulcers represent a very frequent pathology in the Western world, mainly affecting the elderly, thus determining a high social burden.

**Diabetic ulcers,** in particular those responsible for diabetic foot, occur when the diabetic neuropathy or lower limb arteriopathy compromise the function or the structure of the foot. The two conditions, also defined as neuropathic foot or ischemic foot, are profoundly different from each other: however, in the majority of subjects, especially the elderly, both coexist, leading to the so-called neuroischemic foot.

**Surgical wound** is a disruption of tissue continuity caused by a mechanic agent. In clinical practice, two main types of surgical wounds can be encountered:
- wounds that heal by primary intention, wherein the edges have been brought together by suturing. They heal quickly, typically developing a straight scar i.e., often barely visible;
- wounds that heal by secondary intention, wherein the edges are not brought together often due to an infection. Healing is slow, and the scar that forms may vary in size.

**Cut wounds** are one of the most common household accidents and are caused by sharp objects such as knifes or glass. They typically appear as linear wounds and with clear edges.

**Puncture wounds** are caused by pointed objects (knifes, nails, etc.) which cause lesions more developed in depth than on the surface. In addition to the entry hole, these wounds have a trajectory that extends into depth and can even have characteristics of penetrating or perforating wounds when the presence of an exit hole is also observed.

**Gunshot wounds** are caused by projectiles (bullets or fragments) of various shapes and sizes fired from firearms or produced from the explosion of explosive devices. When the projectile is driven by a very high penetrating force, it may exit the hit body through an exit hole: which is usually larger and more ragged than the entry hole since along its path the projectile carries tissue fragments with it, increasing its dimensions.

**Abrasions** are wounds caused by a superficial damage to the skin, not deeper than the epidermis. They are less severe than lacerations and the bleeding, if present, is minimal. The most common abrasions occur when the skin rubs against a rough surface. They may be distinguished into:
- first-degree abrasions: involve only epidermal damage;
- second-degree abrasions: involve the epidermis and the dermis and they may bleed slightly;
- third-degree abrasions: involve damage to the subcutaneous layer of the skin and they are often referred to as avulsions, when the layers of the skin are fully removed.

**Excoriations** are superficial lesions of the skin of traumatic origin that affect the epidermis and superficial dermis. Excoriations manifest with varying degrees of pain, swelling, redness and irritation of the skin and possible bleeding. Depending on the degree of epidermal removal, two types of excoriations are distinguished:
- first-degree excoriations: the lesion involves the outermost layer of the epidermis. It is a mild-type wound, usually not accompanied by bleeding, with a tendency to heal after a few days;
- second-degree excoriations: the wound is deeper, also involves the dermis and it is often accompanied by bleeding. The wound healing occurs within 5-7 days.

A **burn** is an acute wound of traumatic origin caused by contact with a flame, with hot liquids or solids, chemical substances, electricity or radiation energy. Depending on the depth, they are distinguished into first- and second-degree superficial, second- and third-degree deep burns; the optimal treatment of the first two is dressings that generally allow healing within two weeks; in the case of deep burns, surgical treatment is optimal.

Various substances or products exhibit properties useful for the regeneration of tissues affected by wounds. Further, numerous attempts to combine various substances are known from commercially available products or scientific and patent literature, aiming to achieve effective dressings in promoting tissue repair processes, protecting against infections and maintaining an optimal microenvironment.

Although numerous attempts have been made (from commercially available products or from scientific and patent literature) to combine various substances in order to achieve effective dressings in promoting tissue repair processes, protecting against infections and maintaining optimal moisture and oxygen permeability conditions in the wound microenvironment, there is still a need to be able to combine different substances with different properties, in proportions such as to exhibit synergistic effects in terms of promoting the tissue repair process, protecting against infections, and maintaining optimal moisture and oxygen permeability conditions in the wound microenvironment, while also being well-tolerated and easy to apply.

In fact, there are various technical issues that an ideal advanced dressing should solve. In particular, such dressings should:
- act as vehicle for active substances,
- exhibit very low toxicity/cytotoxicity towards the damaged tissue during healing, but be active against microorganisms, so as to ensure microbial load control,
- be well tolerated, comfortable, and non-painful,
- be suitable also for prolonged and repeated use,
- be easy to apply,
- not require frequent changes,
- not adhere to the tissue of the lesion,
- be readily removable/detachable, or be absorbable,
- preserve wound margins,
- control unpleasant odours which may emanate from the wound,
- allow for gas exchange of oxygen, carbon dioxide, and water vapor with the environment,
- be impermeable to the entry of external microorganisms,
- prevent or treat the infection,
- ensure mechanical protection (protect the lesion from potential trauma),
- possibly, allow the monitoring of the healing process without the need to remove the dressing,
- be compatible with any other medication or treatment to be applied on the wound bed (for example antimicrobial drugs, analgesics, anti-inflammatories, local anaesthetics and the like), with therapies such as those with negative pressure, and with possible secondary dressings used as supplementation or for securing primary dressings.

Overall, they should thus have a good compliance from subjects being treated with them, for example by providing an efficient dose of various active substances that act in synergy, and chemical-physical, mechanical and biocompatibility characteristics such that frequent applications are not required, preferably i.e. being applicable once a day or less frequently.

### SUMMARY OF THE INVENTION

The abovementioned aim has been achieved by an advanced dressing characterized by a powder of polymeric material based on poly(lactic-co-glycolic) acid, functionalized with a composition of active substances comprising or consisting of:
dextran
maltodextrin
collagen
citric acid
chitosan
polyphenols
colloidal silver
hyaluronic acid and/or salts thereof
polyethylene glycol
alginate.

The advanced dressing of the invention is a primary, non-adherent dressing, to be applied to the wound bed, antiseptic, hydrophobic, which can be associated with a secondary dressing such as a non-woven fabric gauze, a cotton gauze and/or a fixing system such as a plaster or a self-adherent bandage and the like.

The inventors of the present invention have surprisingly found that the aforementioned ingredients (dextran, maltodextrin, collagen, citric acid, chitosan, polyphenols, colloidal silver, hyaluronic acid and/or salts thereof, polyethylene glycol, alginate) when incorporated in a powder of polymeric material based on poly(lactic-co-glycolic) acid, show synergistic activity among them in terms of low adhesiveness (making the dressing painless at the time of change/removal), antimicrobic power (protecting against exogenous infections), haemostatic power, stimulation of the tissue regeneration process, maintenance of optimal moisture and oxygen permeability conditions in the wound microenvironment.

An object of the present invention is therefore an advanced dressing characterized by a powder of polymeric material based on poly(lactic-co-glycolic) acid, functionalized with a composition of active substances comprising or consisting of:
dextran
maltodextrin
collagen
citric acid
chitosan
polyphenols
colloidal silver
hyaluronic acid and/or salts thereof
polyethylene glycol
alginate,
as outlined in the appended claims from 1 to 8.

A further object of the invention is a process for producing the advanced dressing, as outlined in the appended claim 9.

A further object of the invention is the advanced dressing for use in treating non-necrotic skin wounds, as outlined in the appended claims 10 and 11.

In fact, lesions that, in particular, can benefit from the application of the advanced dressing of the present invention are non-necrotic skin wounds such as, but not limited to, diabetic ulcers, pressure ulcers and sores (bedsores), general skin ulcers and sores, surgical wounds, cut wounds, puncture wounds, gunshot wounds, abrasions, excoriations, burns and, in general, wounds wherein the depth is such as not to involve the subcutaneous, fascial, muscular, and tendinous planes.

The text of the appended claims forms an integral part of the present description for the purposes of assessing sufficiency of disclosure.

Additional characteristics and advantages of the advanced dressings and of the process according to the invention will emerge from the following description of exemplary embodiments, provided by way of example and not limitation.

### BRIEF DESCRIPTION OF THE DRAWINGS

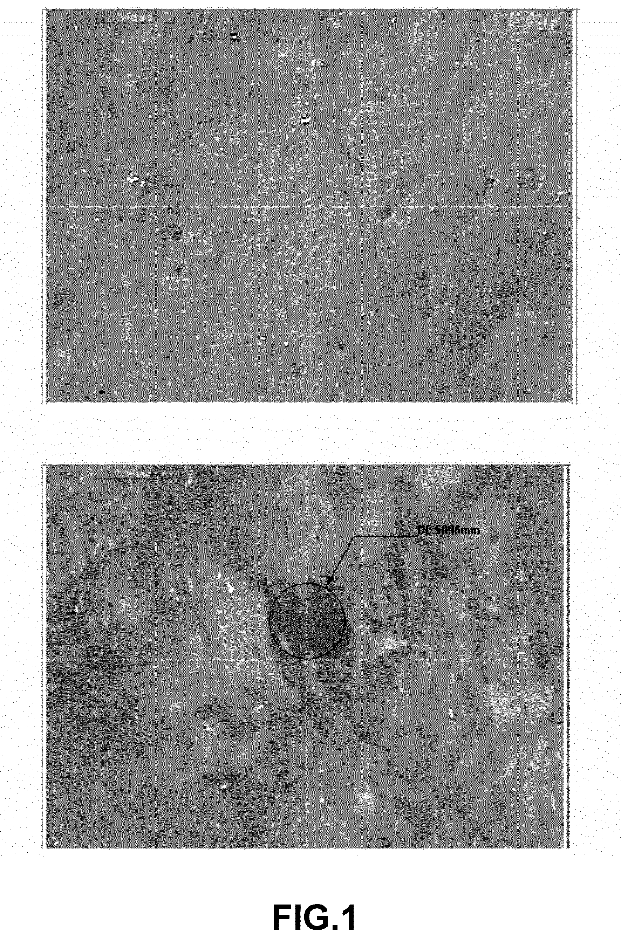

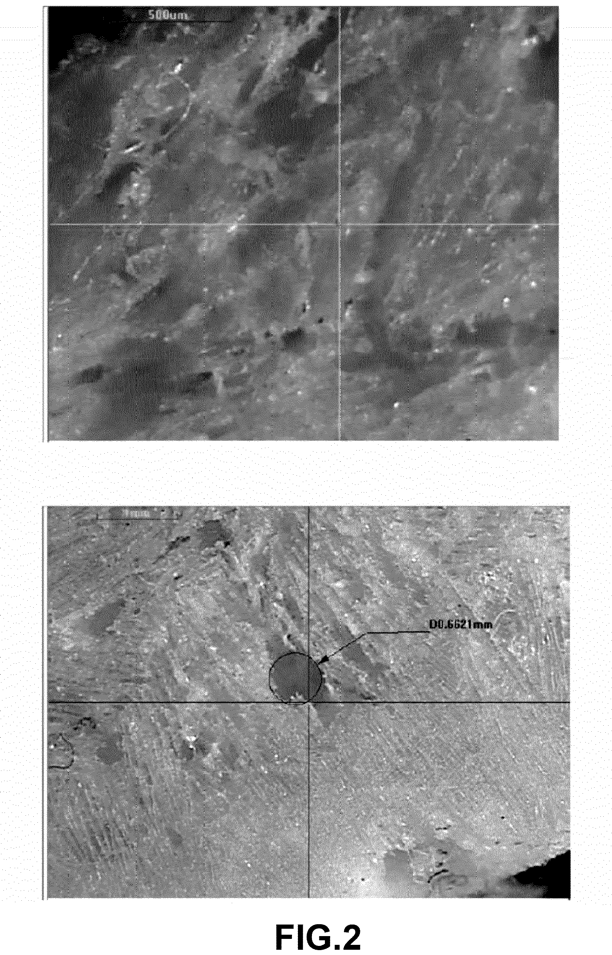

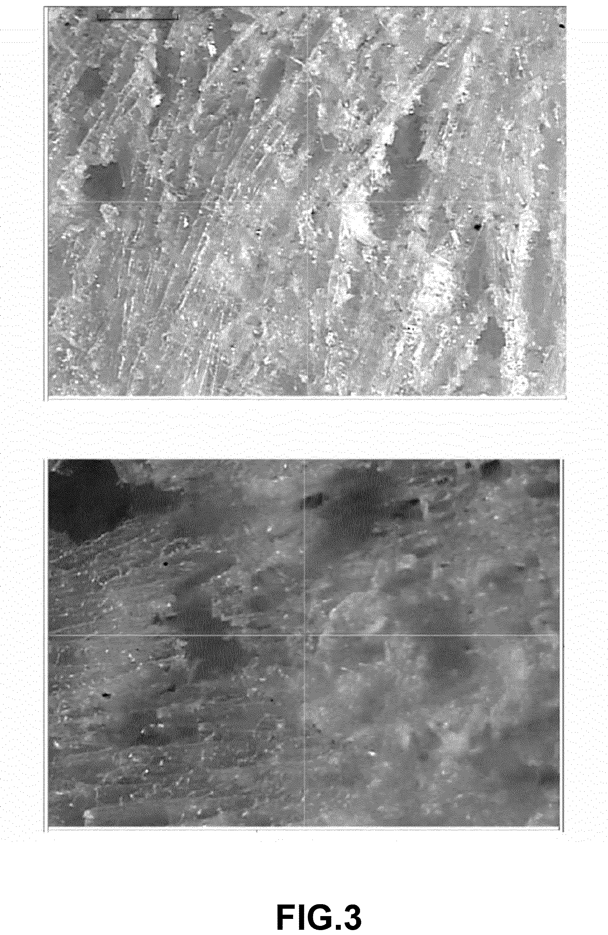

Figures 1-3 show magnified microscope views of an advanced dressing in the form of a functionalized powder of polymeric material, wherein some pores and their sizes are highlighted.

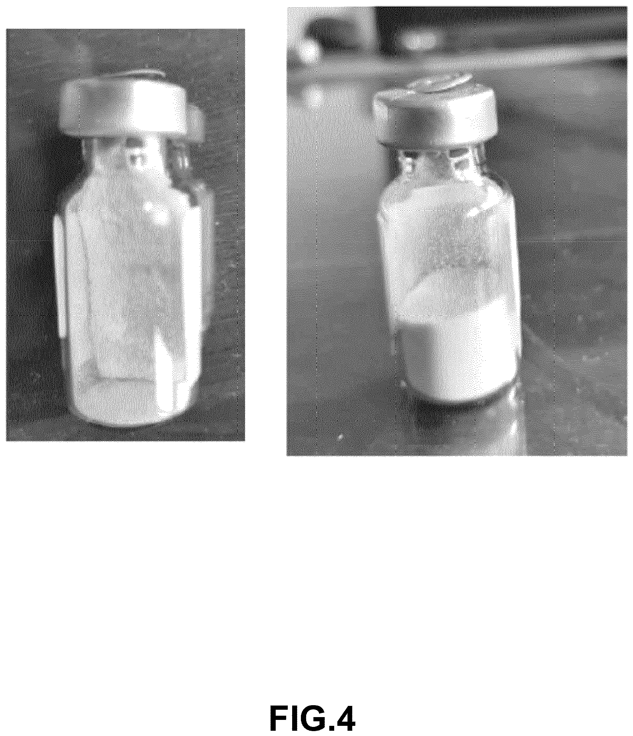

Figure 4 shows an example of packaging for the advanced dressing of the invention in a sterile vial.

### DETAILED DESCRIPTION OF THE INVENTION

According to a first embodiment, the present invention relates to an advanced dressing in the form of a powder of polymeric material based on poly(lactic-co-glycolic) acid, functionalized with a composition of active substances comprising or consisting of:
dextran
maltodextrin
collagen
citric acid
chitosan
polyphenols
colloidal silver
hyaluronic acid and/or salts thereof
polyethylene glycol
alginate.

According to certain embodiments of the invention, the composition of active substances is characterized by the following weight percentages, calculated based on the total weight of the mixture of said components:
40 to 60% w/w dextran
12 to 25% w/w maltodextrin
5 to 10% w/w collagen
5 to 10% w/w citric acid
2 to 10% w/w chitosan
0.05 to 2.5% w/w polyphenols
0.001 to 0.005% w/w colloidal silver
1 to 8% w/w hyaluronic acid and/or salts thereof
1 to 5% w/w polyethylene glycol
1 to 5% w/w alginate.

According to a preferred embodiment of the invention, the composition of active substances is characterized by the following weight percentages, calculated based on the total weight of the mixture of said components:
45 to 55% w/w dextran
15 to 20% w/w maltodextrin
6 to 9% w/w collagen
6 to 9% w/w citric acid
4 to 7% w/w chitosan
0.1 to 1% w/w polyphenols
0.001 to 0.005% w/w colloidal silver
2 to 5% w/w hyaluronic acid and/or salts thereof
2 to 4% w/w polyethylene glycol
2 to 4% w/w alginate.

As used herein, the term "advanced dressing" means an advanced dressing suitable for the treatment of skin wounds. There are primary and secondary dressings: primary ones are positioned and act directly on the wound bed, while secondary ones are used as supplementation, or for securing primary dressings. Non-limiting examples of primary dressings are alginates, hydrocolloids, hydrofibers and polyurethane foams. Non-limiting examples of secondary dressings are non-woven fabric gauzes, cotton gauzes and fixing systems such as plasters or self-adherent bandages.

As used herein, the term "composition of active substances" means a composition of substances that provide a therapeutic effect to the advanced dressing, wherein preferably at least two or more substances have synergistic effects on each other.

As used herein, the term "comprising" means that the composition of active substances described above may contain other active substances, as will be defined in the following, or non-active substances, i.e. substances that do not provide a therapeutic effect to the advanced dressing.

Preferably, the advanced dressing is in the form of a powder of polymeric material based on poly(lactic-co-glycolic) acid, functionalized with active substances capable of promoting the tissue repair process, maintaining optimal moisture and oxygen permeability conditions in the wound microenvironment, preventing the entry of external microorganisms due to the antimicrobial activity and providing haemostatic properties.

Further, the advanced dressing of the invention has physical and mechanical properties such as to make it well-tolerated, comfortable and non-painful, easy to apply and remove (being non-adherent to the affected tissue) or absorbable, non-damaging to the wound margins.

As used herein, the term "poly(lactic-co-glycolic) acid", also known as PLGA, is a copolymer i.e., used in many therapeutic devices approved by the Food and Drug Administration (FDA), thanks to its biodegradability and biocompatibility. It is synthesized by ring-opening copolymerization of two different monomers, the cyclic dimers (1,4-dioxane-2,5-diones) of glycolic acid and lactic acid. During polymerization, successive monomeric units of glycolic or lactic acid are linked together in PLGA by ester bonds, thus obtaining a linear aliphatic polyester as the product. The PLGA degrades by hydrolysis of its ester bonds in the presence of water, producing the original monomers lactic acid and glycolic acid. Under physiological conditions, these monomers are byproducts of various metabolic pathways, and there is no systemic toxicity associated with the use of PLGA for drug delivery applications or in biomaterials. Moreover, it is possible to regulate the degradation time of the polymer by altering the ratio of the monomers used during synthesis. This property makes it suitable and versatile in the production of biomedical devices, such as grafts, sutures, implants, prosthetic devices, surgical sealing films, micro and nanoparticles. In the advanced dressing of the present invention, the poly(lactic-co-glycolic) acid copolymer acts both as a carrier of active substances, regulating their release at the wound site, and to promoting the maintenance of optimal moisture and oxygen permeability conditions in the wound microenvironment. Further, the poly(lactic-co-glycolic) acid copolymer acts as a scaffold for tissue regeneration.

As used herein, the term "powder of polymeric material based on poly(lactic-co-glycolic) acid" means a powder consisting of PLGA functionalized with active substances and intended for use in wounds dressings.

As used herein, the term "dextran" means a variety of branched glucose polymers of variable molecular weight. Dextran is characterized by a succession of D-glucose molecules linked together by an alpha 1-6 glycosidic bond with short branches at alpha 1-2, 1-3 and 1-4 positions. Dextran appears as a white, water-soluble powder and is classified based on molecular weight. It is water-soluble and acts as a binder with the polymer.

As used herein, the term "maltodextrin" means a water-soluble, complex carbohydrate. It is obtained through chemical hydrolysis processes, mainly from the breakdown of starches from cereals (corn, oat, wheat, rice) or tubers (potatoes, tapioca). Depending on the degree of transformation of these starches, maltodextrins are created, consisting of glucose molecules arranged in polymer chains of varying lengths. Maltodextrin typically consists of a variety of chains ranging from 3 to 17 glucose units.

As used herein, the term "collagen" means the most abundant protein in the body, where it is mainly concentrated in bones, tendons, cartilage, skin, membranes and blood vessels. In particular, collagen is one of the major components of connective tissue. In the skin, collagen contributes to maintaining firmness, tone and turgor. It is present in the dermis where, together with elastic fibres and glycosaminoglycans, it forms a three-dimensional structure that supports and sustains the skin, giving it strength and elasticity.

As used herein, the term "citric acid" means 2-hydroxy-1,2,3-propanetricarboxilic acid. It appears as a solid, colourless substance with molecular formula C₆H₈O₇, water-soluble over a wide pH range. It can be found in fruits, mainly of the *Citrus* genus.

As used herein, the term "chitosan" means a linear polysaccharide consisting of D-glucosamine and N-acetyl-D-glucosamine, linked by ß(1-4) bonds. Chitosan is usually obtained by deacetylation of chitin, generally extracted from the exoskeleton of crustaceans (crabs, shrimp, etc.) with an aqueous solution of sodium hydroxide, or it can be of plant or fungal origin, i.e. obtained by deacetylation of chitin present in fungi, where it constitutes the main component of the cell wall. Characteristics that distinguish various types of chitosan and are responsible for their different properties are viscosity, degree of acetylation and molecular weight. Viscosity is measured in 1% acetic acid aqueous solution and expressed in centipoise (cP). Viscosity is measured on a Brookfield rotational viscometer model NDJ-1 that can be used in a viscosity range from 10 to 100,000 cP. At room temperature, 3.0 g of the sample under examination, previously dried to constant weight at 105±2°C, are taken before being placed in 300 ml of water with stirring. Then, 3.0 g of glacial acetic acid are added and the stirring is continued for 1 h or until the sample is completely dissolved. The rotational viscometer is then used to determine the viscosity at 20±1°C. The degree of acetylation is determined by using NMR spectroscopy and varies between 0% and 40%, relative range of molecular weight varies between 3,800 and 20,000 Dalton.

As used herein, the term "polyphenols" means the total amount of compounds with a polyphenolic structure, expressed as equivalents of caffeic acid and determined analytically by the Folin-Ciocalteu method [Ainsworth EA and Gillespie KM, Estimation of total phenolic content and other oxidation substrates in plant tissues using Folin-Ciocalteu reagent, Nature Protocols 2007;2:875-875]. Non-limiting examples of polyphenols are tannic acid, quercetin, apigenin, resveratrol, isoflavones, catechins. Non-limiting examples of sources of polyphenols are plant extracts such as green tea extract, chestnut extract, acai fruit extract, red grape extract, pomegranate extract, olive leaves extract, etc.

As used herein, the term "colloidal silver" means a compound based on silver particles. It appears as shiny grey-black-blue flakes or granular powder, substantially odourless, insoluble in alcohol and ether, slightly water-soluble.

As used herein, the terms "hyaluronic acid" and "hyaluronic acid and/or salts thereof" mean a non-sulphated glycosaminoglycan, devoid of protein core, with non-branched polysaccharidic chain produced from the condensation of thousands of disaccharide units formed in turn by residues of glucuronic acid and N-acetylglucosamine, linked together alternately by β1→4 and β1→3 glycosidic bonds, as well as by intramolecular hydrogen bonds, which stabilize its conformations. Under physiological pH, the carboxylic groups of the glucuronic units are ionized, conferring high polarity to the hyaluronate molecule and consequently high water solubility. Due to this property, the hyaluronate is capable of complexing with a very high number of water molecules, achieving a high degree of hydration. Hyaluronic acid is one of the fundamental components of human and other mammalian connective tissue. It imparts to the skin its particular properties of strength and maintenance of shape. There are hyaluronic acids of different molecular weights; the latter can vary from 2 to 1,500 kDa. Hyaluronic acids with different molecular weights show different and characteristic viscosity values for the aqueous solutions containing them, as well as different degrees of penetration into the skin. As used herein, the terms "hyaluronic acid" and "hyaluronic acid and/or salts thereof" indicate hyaluronic acids with various molecular weights and/or their salts, such as, but not limited to, sodium hyaluronates, and/or mixtures of hyaluronic acids with various molecular weights and/or their salts.

As used herein, the term "polyethylene glycol", also known as polyethylene glycol (PEG), polyethylene oxide (PEG), or polyoxyethylene (POE), IUPAC name poly(oxy-1,2-ethanediyl),α-hydro-ω-hydroxyethane-1,2-diol, ethoxylate, means a polymer prepared by the polymerization of ethylene oxide. In general, the polyethylene glycol features mean molecular weight values comprised between 300 and 10,000,000 g/mol. The physical properties of the polyethylene glycol (as for example the viscosity) vary depending on the mean length of the macromolecules, i.e., the average number n of repeating units, while the chemical properties remain almost unaltered. For low values of n, the polyethylene glycol is liquid, whereas as n increases, it takes on the appearance of a waxy solid with a relatively low melting point. The absence of toxicity for PEG allows its use in the pharmaceutical field, and in particular for parenteral, topical, ophthalmic, oral and rectal pharmaceutical formulations, for example as hydrophilic bases for ointments, as suspending agents or thickening agents in emulsions, in the preparation of vehicles for parenteral formulations, as plasticizers in the production of films for tablets.

As used herein, the term "polyethylene glycol" indicates polyethylene glycol with any molecular weight value and/or mixtures of different polyethylene glycols with different values of molecular weight.

As used herein, the term "alginate" means a chemical compound consisting of an alginic acid salt. Salts of alginic acid are, but not limited to, sodium salts, calcium salts and potassium salts. Alginate is extracted from cell walls of algae and has the appearance of a rubber. It has numerous uses, both in food industry (as emulsifying, thickening, or gelling agent) and in the pharmaceutical field. The alginate quickly absorbs water, this property makes it useful for absorbing any exudate present in wounds.

The components of the present dressing are all known and commercially available individually.

In certain embodiments, the powder according to the invention comprises or consists of, in its entirety, 15 to 20% by weight of a substrate of poly(lactic-co-glycolic) acid and 80 to 85% by weight of a composition of active substances.

Preferably, the composition of active substances contains 2 to 5% by weight of a mixture of high molecular weight hyaluronic acid or sodium hyaluronate and low molecular weight hyaluronic acid or sodium hyaluronate in a ratio of about 1:1.

Preferably, the polyethylene glycol is PEG 4000.

The advanced dressing of the invention may also include additional ingredients and excipients such as, but not limited to, local anaesthetic active substances, analgesic and anti-inflammatory active substances, antimicrobial active substances, growth factors, cytokines, hormones, vitamins, minerals, plant extracts and oils, and the like.

Local anaesthetic active substances are compounds or substances with local anaesthetic activity of synthetic or natural origin. Non-limiting examples of synthetic local anaesthetic active substances are procaine, chloroprocaine, lidocaine, prilocaine, bupivacaine, ropivacaine, levobupivacaine and the like. Non-limiting examples of local anaesthetic active substances of natural origin are peppermint essential oil, myrrh essential oil, eucalyptus essential oil and the like.

Analgesic and anti-inflammatory active substances are compounds or substances with analgesic and anti-inflammatory activity of synthetic or natural origin. Non-limiting examples of synthetic analgesic and anti-inflammatory active substances are steroidal ones (for example cortisone drugs and the like) and non-steroidal ones

(for example NSAIDS and the like) . Non-limiting examples of analgesic and anti-inflammatory active substances of natural origin are those contained in birch extracts (*Betula pendula*), cloves (*Syzygium aromaticum*), meadowsweet (*Spirea ulmaria*), willow (*Salix alba*), and the like.

Non-limiting examples of antimicrobial active substances are bacteriostatic, bactericidal, fungistatic, fungicidal, virustatic, virucidal, and similar drugs of both synthetic and natural origin. Non-limiting examples of antimicrobial active substances are gentamicin, bacitracin, and the like.

Non-limiting examples of vegetable oils are essential oils with antimicrobial activity, such as tea tree oil (*Melaleuca spp*.), or oils with anti-inflammatory activity, such as copaiba oleoresin *(Copaifera spp.).*

The advanced dressing of the invention is presented in the form of a powder with a particle size comprised between 3 and 15 microns, preferably between 5 and 12 microns and contains pores of 0.3 microns to 0.8 microns in diameter.

### EXAMPLES

### Example 1 - advanced dressing based on poly(lactic-co-glycolic) acid

An advanced dressing in the form of a powder of functionalized polymeric material was prepared using the following ingredients (Table 1):

**Table 1.**

| **RAW MATERIAL** | **Amount (w/w %)** |
|---|---|
| Poly(lactic-co-glycolic) acid copolymer | 17.38 |
| Dextran | 42.62 |
| Maltodextrin | 14.412 |
| Collagen | 6.524 |
| Citric acid | 6.524 |
| Chitosan | 4.344 |
| Tannic acid | 0.184 |
| Colloidal silver | 0.0024 |
| High MW sodium hyaluronate | 1.332 |
| Low MW sodium hyaluronate | 1.332 |
| Polyethylene glycol 4000 (PEG4000) | 2.664 |
| Sodium alginate | 2.664 |

The aforementioned advanced dressing is produced by mixing the raw materials in the quantities indicated in Table 1, placing the mixture in moulds of the desired shape and size, subjecting the mixture in the moulds to freeze-drying and eventually micronizing the block resulting from the freeze-drying until the desired particle size is obtained.

### Example 2 - evaluation of the kinetics of microbial reduction inoculated on the advanced dressing

The aim of the test is to establish whether the product, under its conditions of use, demonstrates efficacy in reducing microbial flora. The activity is assessed by contaminating the samples with a defined inoculum of selected microorganism strains and evaluating the reduction of contamination. The inoculum is performed with multiple Gram positive, Gram negative microbial strains, yeasts and moulds. At different but very close time intervals, the residual microbial load is evaluated for each strain, up to 48 hours after inoculation. The inoculum was performed with five microbial strains at different concentrations, as reported in the following Table 2:

**Table 2.**

| **Strain** | **Inoculum concentration** |
|---|---|
| *Escherichia coli* ATCC 8739 | 2.8 × 10⁵ |
| *Pseudomonas aeruginosa* ATCC 9027 | 2.0 × 10⁵ |
| *Staphylococcus aureus* ATCC 6538 | 2.0 × 10⁵ |
| *Candida albicans* ATCC 10231 | 1.9 × 10⁵ |
| *Aspergillus brasiliensis* ATCC 16404 | 3.5 × 10⁵ |

The concentration of viable cells was determined by the plate count method. The diluent used is a solution of sodium chloride-peptone at pH 7 with the addition of some agents for the neutralization of preservatives (polisorbate 80, soy lecithin, L-histidine). Microorganism counts at different timepoints was carried out by taking 1 g of product and diluting it up to 1×10⁶ times. Each dilution was then plated on Petri dishes containing the agarized selective culture medium. The plates were incubated in a thermostat a 34±1°C (for bacteria) or at 22±1°C (for yeasts and moulds) for a time sufficient to allow for a sufficient growth of the microorganism for the purposes of the count (18-24 h for bacteria, 3-7 days for yeasts and moulds). Correcting the number of observed colonies by the dilution factor yields the CFU (Colony Forming Units) value per gram of product.

In addition to the seeding at time zero, subsequent seedings are carried out for the purposes of evaluating the regression curve of the microbial reduction (for bacteria after 10 minutes, 2 hours, 4 hours; for yeasts and moulds after 30 minutes, 4 hours, 8 hours, 24 hours, and 48 hours).

The results are summarized in the following Tables 3-6.

**Table 3. Total microbial counts at different analysis timepoints.**

| *Time* | *Escherichia coli CFU*/*g* | *Pseudomonas aeruginosa CFU*/*g* | *Staphylococcus aureus CFU*/*g* | *Candida albicans CFU*/*g* | *Aspergillus brasiliensis CFU*/*g* |
|---|---|---|---|---|---|
| *Inoculum* | *2.8* × *10⁶* | *2.0* × *10⁶* | *2.0* × *10⁶* | *1.9* × *10⁶* | *3.5* × *10⁶* |
| 10 min | 1.0 × 10⁵ | 1.8 × 10⁴ | 1.0 × 10⁵ | ------- | ------- |
| 30 min | ------- | ------- | ------- | 5.6 × 10⁴ | 4.1 × 10⁴ |
| 2h | <10 | <10 | 1.4 × 10² | ------- | ------- |
| 4h | <10 | <10 | 90 | 1.6 × 10⁴ | 6.0 × 10³ |
| 8h | ------- | ------- | ------- | 1.1 × 10⁴ | 6.0 × 10³ |
| 24h | ------- | ------- | ------- | 1.0 × 10⁴ | 1.0 × 10³ |
| 48h | ------- | ------- | ------- | 1.0 × 10⁴ | 8.3 × 10² |

**Table 4. Logarithmic reduction of microbial count.**

| *Time* | *Escherichia coli CFU*/*g* | *Pseudomonas aeruginosa CFU*/*g* | *Staphylococc us aureus CFU*/*g* | *Candida albicans CFU*/*g* | *Aspergillus brasiliensis CFU*/*g* |
|---|---|---|---|---|---|
| 10 min | 0.45 | 1.05 | 0.30 | ------- | ------- |
| 30 min | ------- | ------- | ------- | 0.53 | 0.67 |
| 2h | >4.45 | >4.30 | 3.15 | ------- | ------- |
| 4h | >4.45 | >4.30 | 3.35 | 1.07 | 1.50 |
| 8h | ------- | ------- | ------- | 1.24 | 1.50 |
| 24h | ------- | ------- | ------- | 1.28 | 2.28 |
| 48h | ------- | ------- | ------- | 1.28 | 2.36 |

### Example 3 - Evaluation of polyphenol release from an advanced dressing in the form of a powder of functionalized polymeric material

The aim of the present study was to evaluate the release of polyphenols over time from an advanced dressing in the form of a powder of functionalized polymeric material.

A dressing weighing 1.5 g was placed in a crystallizer and wetted with 20 ml of water. At the timepoints indicated below, a 50 microliter sample was taken from the aqueous solution in contact with the dressing, and the content of total polyphenols released over time was evaluated on this sample. This evaluation was performed using the Folin-Ciocalteau assay. The timepoints were the following: 10 min, 30 min, 2 hours, 4 hours, 8 hours, 24 hours, 48 hours.

For the execution of the assay, the samples were placed in wells of transparent microplates. In row A, a solution of gallic acid was placed as a positive control, with 1:1 serial dilutions going from well A1 to well A6. In rows B to H, samples taken at different timepoints were placed, also subjected to 1:1 serial dilutions going from well 1 to well 6. Regarding the concentrations of gallic acid (expressed in mg/ml), see Table 5 shown below.

**Table 5.**

| | 1 | 2 | 3 | 4 | 5 | 6 |
|---|---|---|---|---|---|---|
| A (gallic acid solution, as positive control), mg/ml | 0.15 | 0.075 | 0.0375 | 0.01875 | 0.009375 | 0.0046875 |

The results are summarized in Table 6 below, where the estimated concentrations of total polyphenols (x) released by the advanced dressing at various timepoints are expressed as mg/ml of gallic acid.

**Table 6.**

| **Time** | **Concentrations of total polyphenols released** |
|---|---|
| t = 10 minutes | 0.0046875<x<0.009375 |
| t = 30 minutes | 0.0046875<x<0.009375 |
| t = 2 hours | 0.0046875<x<0.009375 |
| t = 4 hours | x=0.009375 |
| t = 8 hours | x=0.009375 |
| t = 24 hours | x=0.009375 |
| t = 48 hours | x=0.009375 |

### Example 4 - Application of the advanced dressing on a wound

Hereinafter is reported an exemplary procedure for the application of the dressing.
1) Preparing the wound following a standard protocol or according to the practician's instructions.
2) Cleansing the wound by irrigating with a sterile saline solution. If necessary, removing foreign material, cellular debris, and devitalized tissue from the wound using suitable instruments, or with the aid of the mechanical action of a jet of sterile saline.
3) Thoroughly drying the intact skin surrounding the wound, without touching the injured skin. If necessary, shaving the hair around the wound.
4) Applying the powder on the wound bed.
5) Subsequently, applying any secondary covering dressing on the wound, ensuring that the back is inspectable in order to ascertain the degree of exudate saturation, thereby determining the timing for changing the primary dressing and the secondary dressing.

## Claims

1. An advanced dressing in the form of a powder based on poly(lactic-co-glycolic) acid functionalized with a composition of active substances comprising or consisting of:
dextran
maltodextrin
collagen
citric acid
chitosan
polyphenols
colloidal silver
hyaluronic acid and/or salts thereof
polyethylene glycol
alginate.

2. The advanced dressing in the form of a powder according to claim 1, wherein the composition of active substances is **characterized by** the following weight percentages, based on the total weight of the dressing:
40 to 60% w/w dextran
12 to 25% w/w maltodextrin
5 to 10% w/w collagen
5 to 10% w/w citric acid
2 to 10% w/w chitosan
0.05 to 2.5% w/w polyphenols
0.001 to 0.005% w/w colloidal silver
1 to 8% w/w hyaluronic acid and/or salts thereof
1 to 5% w/w polyethylene glycol
1 to 5% w/w alginate.

3. The advanced dressing in the form of a powder according to claim 1, wherein the composition of active substances is **characterized by** the following weight percentages, based on the total weight of the dressing:
45 to 55% w/w dextran
15 to 20% w/w maltodextrin
6 to 9% w/w collagen
6 to 9% w/w citric acid
4 to 7% w/w chitosan
0.1 to 1% w/w polyphenols
0.001 to 0.005% w/w colloidal silver
2 to 5% w/w hyaluronic acid and/or salts thereof
2 to 4% w/w polyethylene glycol
2 to 4% w/w alginate.

4. The advanced dressing in the form of a powder according to any one of claims 1-3, wherein the polyphenols are tannic acid.

5. The advanced dressing in the form of a powder according to any one of claims 1-4, wherein the polyethylene glycol is polyethylene glycol 4000 (PEG4000) and/or the hyaluronic acid and/or salts thereof is a mixture of about 1:1 low molecular weight and high molecular weight hyaluronic acid and/or sodium hyaluronate.

6. The advanced dressing in the form of a powder according to any one of claims 1-5, wherein the powder has a particle size between 5 and 12 microns and contains pores of 0.3 micron to 0.8 micron in diameter.

7. The advanced dressing in the form of a powder according to any one of claims 1-6 further comprising active substances selected from the group consisting of: local anaesthetic agents, analgesic and anti-inflammatory agents, antimicrobial agents, growth factors, cytokines, hormones, vitamins, minerals, plant extracts and oils, and the like.

8. The advanced dressing according to any one of claims 1-7, in the form of a powder, wherein the powder comprises or consists of, in its entirety, 15 to 20% by weight of a substrate of poly(lactic-co-glycolic) acid and 80 to 85% by weight of the composition of active substances.

9. A process for producing an advanced dressing in the form of a powder according to any one of claims 1-8, said process comprising the following steps:
- preparing the mixture containing the copolymer and the other active substances, ingredients and excipients
- placing the mixture in moulds of the desired shape and size
- freeze-drying the mixture in the moulds
- micronizing the freeze-dried mixture until the desired particle size is obtained
- sterilizing and packaging.

10. The advanced dressing in the form of a powder according to any one of claims 1 to 8, for use in treating non-necrotic skin wounds.

11. The advanced dressing in the form of a powder for use according to claim 10, wherein the non-necrotic skin wounds are selected from the group consisting of: diabetic ulcers, pressure ulcers and sores (bedsores), general skin ulcers and sores, surgical wounds, cut wounds, puncture wounds, gunshot wounds, abrasions, excoriations, burns.
